(19) **Europäisches Patentamt**
**European Patent Office**
**Office européen des brevets**

(11) **EP 1 395 524 B1**

(12) **EUROPÄISCHE PATENTSCHRIFT**

(45) Veröffentlichungstag und Bekanntmachung des Hinweises auf die Patenterteilung:
**21.02.2007 Patentblatt 2007/08**

(21) Anmeldenummer: **01965227.0**

(22) Anmeldetag: **29.08.2001**

(51) Int Cl.:
***C04B 14/00*** *(2006.01)*

(86) Internationale Anmeldenummer:
**PCT/EP2001/009951**

(87) Internationale Veröffentlichungsnummer:
**WO 2002/018292 (07.03.2002 Gazette 2002/10)**

(54) **VERBESSERTE QUELLFÄHIGE SCHICHTSILIKATE**

IMPROVED SWELLABLE PHYLLOSILICATES

SILICATES EN COUCHE EXPANSIFS AMELIORES

(84) Benannte Vertragsstaaten:
**AT BE CH CY DE DK ES FI FR GB GR IE IT LI LU MC NL PT SE TR**

(30) Priorität: **29.08.2000 DE 10042455**

(43) Veröffentlichungstag der Anmeldung:
**10.03.2004 Patentblatt 2004/11**

(73) Patentinhaber: **SÜD-CHEMIE AG**
**80333 München (DE)**

(72) Erfinder:
• **MÖLLER, Markus**
**80804 München (DE)**

• **COUTELLE, Helmut**
**85356 Freising (DE)**
• **WARTH, Robert**
**85368 Moosburg (DE)**
• **HEININGER, Wolfgang**
**85368 Moosburg (DE)**

(74) Vertreter: **Splanemann Reitzner Baronetzky Westendorp**
**Rumfordstrasse 7**
**80469 München (DE)**

**Beschreibung**

Die Erfindung betrifft verbesserte quellfähige Schichtsilicate auf Smektitbasis zur Verwendung in die Quellfähigkeit herab setzenden Medien.

Die Verwendung von quellfähigen Schichtsilicaten auf Smektitbasis, z.B. der Bentonite, Montmorillonite, Hectorite, usw., z.B. in mineralischen Bindemittelsystemen, in Farben und/oder Lacken, Kosmetika, Schmierfetten und einer Vielzahl anderer Anwendungen ist bekannt.

Generell ist hierbei zu beachten, dass die für diesen Zweck brauchbaren Schichtsilicate bestimmte Voraussetzungen erfüllen müssen, die von der jeweiligen Anwendung abhängen. So dürfen z.B. in zementären Systemen keine negativen Erscheinungen auftreten, wie z.B. Rissbildung oder Schwund. So wird z.B. von Björn Lagerblad und Berit Jacobson in "Proc. Int. Conf. Cem. Microsc. (1997) (19th ed., S. 151 - 162) darauf hingewiesen, dass die Verwendung von quellfähigen Smektiten in Zement und/oder Beton zu Problemen mit Rissbildung führt.

In der EP-A-0 675 089 wird eine Möglichkeit beschrieben, wie dieses Problem umgangen werden kann und die quellfähigen Schichtsilicate dennoch in zementösen mineralischen Bindemittelsystemen verwendet werden können. Das in dieser Druckschrift beschriebene Hilfsmittel für mineralische Bindemittelsysteme besteht zu mindestens 60% aus einem quellfähigen Schichtsilicat mit einem Quellvolumen von etwa 5 bis 50 ml, bezogen auf eine Suspension von 2g Schichtsilicat in 100 ml Wasser. Die Feinheit des Schichtsilicats ist nicht ausdrücklich erwähnt; auf Seite 3, Zeile 32 wird aber ein "typischer" maximaler Siebrückstand von etwa 20 Gew.-% auf 90 μm erwähnt. Eine wesentliche Lehre dieser Druckschrift besteht darin, dass Bentonite innerhalb eines speziellen Quellvolumens einen geeigneten Kompromiss darstellen, bei dem einerseits noch eine gewisse Plastifizierung auftritt, andererseits aber noch nicht die Gefahr besteht, dass die ausgehärteten Systeme Schwundrisse aufweisen.

In vielen anderen Trockenmörtelsystemen, die ebenfalls in der Bauindustrie Verwendung finden, ist das Quellvermögen der smektitischen Schichtsilicate allerdings nicht der limitierende Faktor: In diesen Systemen auf Basis z.B. von Gips oder Kalk/Gips-Kombinationen werden quellfähige Schichtsilicate meist schon deswegen nicht eingesetzt, weil die "potentielle" Wirkung dieser Materialien nicht entsprechend zur Geltung kommt.

So stellt Alther in "Applied Clay Science", I (1986) Seiten 273 bis 284 klar, dass die Anwesenheit von Gips selbst in kleineren Anteilen die rheologischen Eigenschaften von Bentoniten beeinträchtigt.

Diese "potentielle" Wirkung der Produkte beruht einerseits auf der ausgeprägten Schichtstruktur und andererseits auf deren Quellfähigkeit in Wasser und der damit verbundenen hohen Oberfläche. Durch die Schichtstruktur bedingt, können die Schichtsilicate als internes Schmiermittel wirken, sobald die Lamellen durch Einquellen von Wasser voneinander getrennt werden und übereinander gleiten können. Dadurch werden die im Normalfall hochgefüllten Systeme wesentlich geschmeidiger und sind leichter pumpbar. Damit wird eine Vielzahl von Anwendungen verbessert, wie z.B. die Pumpbarkeit der gebrauchsfertigen Systeme, das manuelle Auftragen von z.B. ansonsten zähen Systemen, wie Fliesenkleber, Mauermörtel, das "Verziehen" von Putzen oder das Auftragen von Edelputzen, die Reduzierung der Sedimentation von (groben) Zuschlagstoffen in fließfähigen Systemen (Fließestriche und Verfüllmörtel) usw.

Diese potentielle Wirkung der quellfähigen Schichtsilicate kann aber nur bedingt ausgenützt werden, da aufgrund ihrer Natur der Einsatz in Systemen, die eine größere Menge an löslichen Kationen, insbesondere an zweiwertigen Kationen, wie Ca-Ionen, enthalten, begrenzt ist. Die üblicherweise verwendbaren Schichtsilicate, wie Bentonite, Montmorillonite, Hectorite usw. sind kolloidale Systeme, die zwischen den Lamellen einwertige Kationen enthalten. Nur wenn deren Hydratationsenergie höher ist als die Anziehung der negativ geladenen Lamellen durch diese Kationen, quellen diese Materialien in Gegenwart von Wasser auf. Besonders geeignet sind daher Smektite mit Natriumionen zwischen den Schichten. Aufgrund der Ladungsdichte der anderen Alkali-Ionen sind Li- oder K-Smektite schon wesentlich weniger quellfähig als die entsprechenden Na-Smektite.

Bei Zugabe und/oder Anwesenheit von zweiwertigen Kationen flocken diese Materialien in der Regel aber schnell wieder aus, da durch Ionenaustausch aus den quellfähigen Schichtsilicaten nur noch bedingt und innerkristallin "anquellende" Schichtsilicate entstehen.

Werden aus Gründen der einfachen Handhabung nun pulverförmige und quellfähige Schichtsilicate in einer Pulvermischung eingesetzt, die als wesentliche Bestandteile u.a. hydraulische oder latent-hydraulische Bindemittel enthält, die in Gegenwart von Wasser relativ große Mengen an z.B. zweiwertigen Ca-Ionen freisetzen, so tritt umgehend eine Konkurrenzreaktion zwischen der Wassereinlagerung zwischen die Schichtsilicatlamellen (Quellen) und der gegenläufigen Flockung auf. Beim Zumischen eines Schichtsilicat-Pulvers zu einem ebenfalls pulverförmigen Bindemittelsystem, das zudem reich an wasserlöslichen Ca-Ionen ist, wird das Schichtsilicat bei Zugabe von Wasser zu der Pulvermischung gleich von Anfang am Aufquellen gehindert, so dass die potentielle Wirkung auch gleich von Anfang an nicht vollständig zum Tragen kommt.

Die Effizienz der Produkte entspricht daher im Normalfall nur einem Bruchteil ihrer "theoretischen" Wirkungsmöglichkeit, da ein Teil der Wirkung durch Einlagerung der zweiwertigen Kationen und "Ausflockung" bei Anwesenheit von Ca-Ionen wieder zunichte gemacht wird. Üblicherweise wird daher das quellfähige Schichtsilicat in Form einer vordispergierten Suspension eingesetzt, da dann zumindest sichergestellt ist, dass das Material schon vollkommen aufgequollen ist und eine Einschränkung der Wirksamkeit erst durch nachträgliches Flocken erfolgt.

Diese "zweikomponentige" Arbeitsweise ist aber umständlich und ist zudem nicht in allen Fällen durchführbar, da gut aufgequollene Schichtsilicate, die noch pumpbar sein sollten, um ein Dosieren zu erleichtern, nur in einer Feststoff-Konzentration von etwa 5 bis 15 Gew.-% herstellbar sind. In vielen Einsatzbereichen ist aber die Zugabe von größeren Wassermengen, wie sie durch Suspensionen oder Pasten mit so niedrigem Feststoffgehalt eingetragen werden, nicht akzeptabel. Immer wieder wurden daher Anstrengungen unternommen, entsprechende Suspensionen zur Verfügung zu stellen, bei denen durch Zugabe von Netz- und/oder Dispergierhilfsmitteln zu der Smektitsuspension der Feststoffgehalt auf zumindest > 20 Gew.-% - unter Beibehaltung einer guten Pumpbarkeit - angehoben werden kann, um diesen Nachteil zu umgehen.

So beschreibt die WO 92/11218 eine Methode, Bentonitsuspensionen in höher konzentrierter Form anzubieten. Ziel ist die Bereitstellung von stabilen Suspensionen mit dennoch niedriger Viskosität. Gemäß dieser Druckschrift lassen sich gut pumpbare Suspensionen mit bis zu 50 Gew.-% von quellfähigen Smektiten dadurch herstellen, dass das quellfähige Schichtsilicat nicht in Wasser, sondern in eine 3 bis 15%-ige NaCl-Lösung eingearbeitet wird. Beim Verdünnen mit Wasser kommen die üblichen Eigenschaften des Bentonits zum Tragen.

Eine analoge Thematik ist in der WO 95/09135 beschrieben. Auch hier geht es um die Herstellung von höher konzentrierten Bentonitsuspensionen, was mit Hilfe von Aminsalzen von Aminen mit niedrigem Molekulargewicht erreicht werden kann. Es ist angegeben (Seite 4, Zeilen 8 bis 11), dass alle mehrwertigen Kationen die Neigung haben, die Tonplättchen fest aneinander zu binden, was die Dispergierung beeinträchtigt. Dies zeigt deutlich, dass ein Einsatz von Bentoniten in einer Umgebung, die mehrwertige Kationen enthält, zumindest zweifelhaft ist. Dies gilt übertragen auch in Systemen, die, als Pulver in Wasser eingearbeitet, ebenfalls hohe Salzkonzentrationen ergeben können.

Obwohl damit schon eine Möglichkeit besteht, die entsprechenden Schichtsilicate auch in den kritischen Systemen einzusetzen, weist diese Methode aber einige gravierende Nachteile auf:

* Die Schichtsilicate müssen separat aufbereitet werden, was umständlich ist und weitere Kosten verursacht und zusätzliche Maschinen erfordert.

* Die pastösen bzw. wässrigen Systeme können naturgemäß nicht in Pulvermischungen eingesetzt werden.

* Die Halbfabrikate (Suspensionen) enthalten zusätzliche Salze, die nicht immer erwünscht sind und/oder in das System passen (ökologische Verträglichkeit, Ausblühneigung usw.).

* Die so hergestellten Schichtsilicatdispersionen ergeben die gewünschten Eigenschaften erst nach weiterem Verdünnen mit Wasser, das zumindest bei den zementären oder auf Gips basierenden Baustoffsystemen nicht möglich ist.

Eine ähnliche Thematik wird in der US-A-5,389,146 beschrieben. Um die hohe Viskosität zu vermeiden, die beim Einarbeiten eines feinteiligen Schichtsilicats, wie Bentonit, entsteht und das Pumpen der Systeme (beschrieben werden Dicht- und Verfüllmassen mit 80% Bentonit) erschwert, wird ein granuliertes Material mit viskositätsbrechenden Zusätzen verwendet.

Die Schwierigkeiten, dass das quellfähige Schichtsilicat durch das System, in das es eingearbeitet werden soll, am Aufquellen und Ausbilden der optimalen rheologischen Eigenschaften gehindert werden kann, können analog auch dann auftreten, wenn das System nicht eine zementhaltige Pulvermischung ist, sondern wenn das Material z.B. in eine Tensidlösung eingemischt wird. Im Normalfall unterbindet die Tensidadsorption an der Schichtsilicatoberfläche ein Quellen des Schichtsilicats ebenso wie die Anwesenheit von Kationen, wie Ca-Ionen usw.

In der US-A-2,036,617 wird die Teilchengröße von Bentoniten und der Einfluss dieser Teilchengröße auf die Dispergierbarkeit in Wasser angesprochen. Es wird davon ausgegangen (Seite 1, Spalte 2 und Zeilen 43 ff), dass man früher angenommen habe, dass die Bentonitteilchen um so besser dispergierbar seien, je feiner sie sind. Die Erfinder haben dagegen festgestellt, dass in gewissen Fällen gröbere Bentonitteilchen besser dispergierbar sind als feinere Teilchen. Die beste Wirkung wird daher angenommen, wenn die trockenen Teilchen ungefähr gleiche Durchmesser und eine bestimmte bevorzugte Größe haben. Die kleinsten Partikelgrößen liegen bei etwa 53 $\mu m$ (250 mesh). Außerdem geht es nach dieser Patentschrift nur um die Dispergierung bzw. Dispergierbarkeit in Wasser und nicht um die Beibehaltung eines möglichst hohen Wirksamkeitsanteils bei der Verwendung in Pulversystemen, die bei Kontakt mit Wasser mehrwertige Kationen freisetzen können.

Die DE-A-195 27 161 beschreibt ein Pigmentgemisch und eine Streichfarbe mit verbesserter Tiefdruckeignung, enthaltend ein feinteiliges Calciumcarbonat und mindestens ein feinteiliges quellfähiges Schichtsilicat und/oder ein sauer aktiviertes Schichtsilicat. Es wird nicht unterschieden zwischen einem quellfähigen Schichtsilicat und einem quellfähigen Material, die beide hinsichtlich der Verbesserung der Tiefdruck-Bedruckbarkeit als gleichwertig bezeichnet sind. Die genannten Pigmente ($CaCO_3$ bzw. quellfähiges - oder gleichwertig- saure Schichtsilicat) weisen die für die Papierbeschichtung übliche Einheit auf. Ein übermäßig hohes Quellvermögen ergibt jedoch Streichfarben mit zu hoher Viskosität, wobei beim Einrühren eines quellfähigen Schichtsilicats in eine Calciumcarbonat-Dispersion ein sauer aufgeschlossenes Schichtsilicat bevorzugt wird. Der Druckschrift ist nicht entnehmbar, dass das Schichtsilicat zur Verwendung in einem die Quellfähigkeit herabsetzenden Medium verwendet werden soll.

Die DE-A-44 38 305 betrifft ein Pigment zum Streichen von Druckpapieren, insbesondere ein Farbentwicklerpigment für Selbstdurchschreibepapiere. Die Farbentwicklungseigenschaften werden durch eine saure Aktivierung eines Alkali- und/oder Erdalkalismektits oder durch Belegung oder Aktivierung mit Lewis-Säuren erzielt. Ziel ist es, für die Farbentwicklung genügend reaktive Zentren zur Verfügung zu stellen, ohne die BET-Oberfläche des entstehenden Produkts zu hoch werden zu lassen, da sonst zu viel Bindemittel für die Papierbeschichtung nötig wird. Die Druckschrift enthält keine Hinweise auf eine besondere Feinheit des Materials und die Verwendung des Materials in einem Medium, das die Quellfähigkeit herabsetzt.

Die DE-A-44 13 672 betrifft ebenfalls einen Farbentwickler für Selbstdurchschreibepapiere auf der Basis eines quellfähigen Schichtsilicats, das dadurch gekennzeichnet ist, dass der Anteil des quellfähigen Schichtsilicats 50 bis 100 Gew.-% beträgt und dass das Schichtsilicat ein Quellvolumen von 5 bis 30 ml, bezogen auf eine Suspension von 2g in 100 ml Wasser, und eine spezifische Oberfläche von > 140 $m^2$/g aufweist. Es ist die gezielte weitere Zumischung von Extenderpigmenten, wie Silicaten oder billigen Füllstoffen, wie Kaolin oder Calciumcarbonat erwähnt; es findet sich jedoch kein Hinweis auf eine besonders vorteilhafte Feinheit. Die Farbentwickler werden auch nicht in einem Medium, das die Quellfähigkeit herabsetzt, verwendet.

Die DE-A-42 17 779 betrifft ein Streichpigment zur Beschichtung von Druckträgern, insbesondere Papier und Karton, das mindestens ein quellfähiges Schichtsilicat enthält, welches im wesentlichen ohne Bindemittel auf den Druckträger fixierbar ist, wobei der Anteil des quellfähigen Schichtsilicats mindestens 30 Gew.-% und das Quellvolumen des Streichpigments 5 bis 28 ml, bezogen auf eine Suspension von 2g Streichpigment in 100 ml Wasser, beträgt. Die Druckschrift enthält jedoch keinen Hinweis auf das Verhalten des Streichpigments in einem Medium, das die Quellfähigkeit herabsetzt.

Die DD-A-219 170 betrifft ein Verfahren zur Aktivierung von Rohtonen. Sie enthält keinen Hinweis darauf, dass das Material in einem Medium, das die Quellfähigkeit herabsetzt, verwendet werden soll.

Die DE-C 108 143 betrifft ebenfalls lediglich ein Verfahren zur Herstellung von hochquellfähigen Stoffen aus Tonmineralien, wobei die Aktivierung in Verbindung mit Alkali-Ionen, insbesondere Na-Ionen, unter Zusatz von Magnesiumsalzen durchgeführt wird.

Die CH-A-459 858 betrifft ein Zusatzmittel für Mischungen auf Zementbasis und ein Verfahren zu seiner Herstellung. Das Zusatzmittel wird einer Mischung aus Zement und Sand mit einer bestimmten Kornverteilung zugesetzt und enthält mindestens eine zur Montmorillonit-Gruppe gehörende Substanz, mindestens eine Füllkomponente mit hydraulischen Eigenschaften sowie mindestens einen Entspanner und mindestens einen Abbinderegler. Die zur Montmorillonit-Gruppe gehörende Substanz ist nicht näher spezifiziert, außer dass sie in einer bestimmten prozentualen Menge enthalten sein soll. Der bevorzugt verwendete Bentonit kann auch durch einen organophilen Bentonit ersetzt werden. Die Feinheit der gesamten Zusatzmittelmischung liegt bei 8 bis 10 $\mu$m.

Der vorliegenden Erfindung lag die Aufgabe zugrunde, quellfähige Schichtsilicate auf Smektitbasis zur Verwendung in die Quellfähigkeit herabsetzenden Medien bereitzustellen. Zu diesen Medien gehören beispielsweise pulverförmige mineralische Bindemittelsysteme oder andere pulverförmige und wasserlösliche Salze enthaltende Systeme oder Tensidlösungen. Generell umfasst dies auch alle wässrigen Systeme, die Komponenten enthalten, die das Quellen behindern können. Auch wenn die quellfähigen Schichtsilicate nicht als vorgequollene Suspension eingesetzt werden, sollen sie gleichzeitig die potentielle Wirkung ganz oder überwiegend beibehalten. Eine spezielle Aufgabe bestand darin, quellfähige Schichtsilicate bereitzustellen, die bei der Einarbeitung in Suspensionen und/oder Lösungen mit zwei- oder mehrwertigen Kationen (z. B. Suspensionen und/oder Lösungen von Calciumhydroxid bzw. gelöschtem Kalk (wie z.B. Kalkmilch), Calciumcarbonat oder ähnlichen calciumreichen Verbindungen) dennoch eine Stabilisierung und Viskositätserhöhung ergeben.

Überraschenderweise wurde gefunden, dass bereits eine sehr feine Zerteilung der Schichtsilicate die gewünschte Wirkung ergibt. Dies ist um so überraschender, als man erwarten konnte, dass bei einem sehr feinteiligen Schichtsilicat und der damit erhöhten freiliegenden Oberfläche in Anwesenheit der in Wasser ebenfalls schnell gelösten Ca-Ionen diese Oberfläche sogar noch schneller durch diese zweiwertigen Ionen belegt und die Quellung damit noch schneller und effektiver unterbunden werden würde.

Gegenstand der Erfindung ist somit ein quellfähiges Schichtsilicat auf Smektit- und/oder Sepiolith/Palygorskit-Basis zur Verwendung in die Quellfähigkeit herabsetzenden, zwei- oder mehrwertige Kationen enthaltenden Medien, gekennzeichnet durch ein Quellvolumen von etwa 5 bis 50 ml, gemessen durch Zugabe von 2g Schichtsilicat zu 100 ml Wasser, und durch eine Feinheit von maximal 10% > 20 $\mu$m, als Trockensiebrückstand.

Es kann auch der mittlere Korndurchmesser, z. B. in einem Laser-Messgerät (z. B. Malvern) ermittelt werden. Dieser sollte in der Größenordnung von < 15 $\mu$m, bevorzugt < 10 $\mu$m, insbesondere < 5 $\mu$m liegen. Im Gegensatz dazu liegt die Teilchenfeinheit von handelsüblichen Materialien bei einem Siebrückstand von etwa 15% bei 45 $\mu$m bzw. bei

einem mittleren Korndurchmesser von etwa 30 μm.

Die Feinheit von Materialien, die in hydraulisch abbindenden Systemen eingesetzt werden, ist zwar teilweise schon beschrieben; dabei handelt es sich aber entweder überwiegend um die übliche Produktfeinheit der angebotenen pulverförmigen Materialien oder aber um spezielle, nicht die vorliegende Erfindung betreffende Anwendungen.

So wird in der EP-A-O 409 774 ein Produkt beschrieben, das als Verzögerungsmittel für hydraulisch härtende Systeme eingesetzt wird und das zu etwa 30 bis 60 Gew.-% aus Lignosulfonat und/oder Polyphosphat und 60 bis 30 Gew.-% aus einer mineralischen Komponente besteht (mit geringen Zusätzen an einem Tensid). Die mineralische Komponente (z.B. Vermiculit, Bentonit, Montmorillonit oder Perlit) soll dabei einen Rückstand von maximal 20% auf 50 μm aufweisen.

In der US-A-5,588,990 ist ein System beschrieben, bei dem zu einem Puzzolanzement u. a. Feinststoffanteile aus Talkum und/oder Bentonit oder äquivalente Materialien, wie Flugasche, zugegeben werden. Zwischen den Materialien Talkum, (Calcium)-Bentonit oder Flugasche wird nicht unterschieden. Der Einfluss der Kornfeinheit auf das Quellvermögen wurde nicht erkannt.

In der US-A-5,637,144 wird ein Asbest-Ersatzsystem für zementäre Systeme beschrieben. Das System besteht aus einer Kombination von verschiedenen Materialien, wobei u.a. ein hydrophiler Bentonit mit einem Wasser-Retentionsmittel und anderen Additiven gemischt wird. Das feinteilige Material kann dabei auch ein Kaolin, Montmorillonit, Attapulgit, eine Fullererde oder ein Kieselgur sein. Die wesentliche Eigenschaft des Minerals ist ein gewisses Wasseraufnahmevermögen. Die Feinheit der Materialien wird aber nicht näher definiert.

Das Schichtsilicat liegt gemäß der vorliegenden Erfindung vorzugsweise in einer Feinheit von maximal 5% > 10 μm vor.

Unter Schichtsilicat auf Smektit- oder Sepiolith/Palygorskit-Basis wird hier insbesondere ein Schichtsilicat aus der Smektitgruppe und/oder aus der Sepiolith/Palygorskitgruppe, umfassend z.B. Attapulgit und Sepiolith, verstanden.

Vorzugsweise ist das smektitische Schichtsilicat aus der Gruppe Bentonit, Montmorillonit, Hectorit, Saponit, Beidellit, Sauconit natürlichen oder synthetischen Ursprungs oder deren Gemischen, gegebenenfalls mit einem Anteil an schichtförmigen Extendersilicaten, wie Vermiculit, Illit, Glimmer, Pyrophillit, Talk, Kaolin, ausgewählt.

Vorzugsweise beträgt der Gehalt an Montmorillonit im smektitischen Schichtsilicat mehr als etwa 70 Gew.-%, besonders bevorzugt mehr als etwa 80 Gew.-%, insbesondere mehr als etwa 85 Gew.-%.

Das erfindungsgemäße Schichtsilicat kann vorteilhafterweise auch einen Zusatz von pulverförmigen alkalihaltigen Substanzen enthalten.

Bei den pulverförmigen alkalihaltigen Substanzen handelt es sich z.B. um pulverförmige Wassergläser, Komplexbildner, wie Phosphate, Zeolithe oder andere Materialien, die wie Ionenaustauscher die löslichen Ca-Ionen des Bindemittelsystems vorübergehend "immobilisieren" und das Schichtsilicat in der quellfähigen Form lange genug erhalten, so dass in Wasser die Lamellen getrennt werden und die Quellung des Schichtsilicats eintreten kann.

Gegenstand der Erfindung ist auch ein Verfahren zur Herstellung des erfindungsgemäßen Schichtsilicats, das durch alkalische Aktivierung eines Erdalkali-Schichtsilicats, Trocknen und Vermahlen des Materials mit überlagerter Sichtung gekennzeichnet ist. Die alkalische Aktivierung des Erdalkali-Schichtsilicats erfolgt in an sich bekannter Weise, beispielsweise durch Behandlung einer Suspension des Schichtsilicats mit Soda.

Die alkalische Aktivierung ist auch durch Mischung geeigneter Rohstoffe, wie wenig quellfähigem Ca-Bentonit mit hochquellfähigem Na-Bentonit, möglich. Die Kombination von quellfähigen Schichtsilicaten der Smektit-Gruppe mit anderen, weniger quellfähigen Schichtsilicaten z.B. der Sepiolith-/Palygorskitgruppe ist ebenfalls möglich, um das angestrebte Quellvolumen zu erhalten. Bei der "Aktivierung" wird im Normalfall der erfindungsgemäße Ton immer einem hochscherenden, hochenergetischen mechanischen Vorgang unterworfen, um sicherzustellen, dass der angestrebte Ionenumtausch auch stattfindet und die gewünschte Wirkung im System erkennbar ist. Geeignete Verfahren zur Aktivierung sind dem Fachmann geläufig und umfassen z.B. Kollergänge, Extruder-Verfahren, Walzenstühle, Kolloidmühlen etc. Das optimale Verfahren richtet sich dabei nach dem vorhandenen Rohstoff, der je nach Feuchte und Plastizität relativ wenig Scherenergie benötigt, oder aber ein mehrmaliges Bearbeiten bei hohem Energieeintrag nötig machen kann. Dies kann vom Fachmann anhand routinemäßiger Versuche ermittelt werden. Dies kann soweit gehen, dass der Ton im Wasser suspendiert und in gut pumpbarer Form einer Kolloidmühlenbehandlung unterworfen wird, z.B. einer Behandlung mittels einer Manton-Gaulin-Mühle oder einer Microfluidiser-Behandlung, bei der zwei Suspensionsströme unter hohem Druck nach Art einer JetMill aufeinander geschossen werden und hierdurch die gewünschte Scherenergie eingetragen wird. In diesem Falle einer Aktivierung im "Suspensionszustand" und dem Vorliegen einer gut pumpbaren Suspension kann naturgemäß der Ton auch ausgereinigt und über übliche Trocknungs- und Mahlverfahren hergestellt werden.

Eine Variante des erfindungsgemäßen Verfahrens ist gekennzeichnet durch alkalische Aktivierung eines in einem wässrigen Medium suspendierten Erdalkali-Schichtsilicats, Entfernung der groben Zuschlagstoffe und Sprühtrocknung der verdünnten Suspension, sowie gegebenenfalls durch Mahlung und Sichtung der sprühgetrockneten Teilchen, falls diese die vorstehend angegebene Kornfeinheit noch nicht erreicht haben.

Bei den groben Zuschlagstoffen handelt es sich z. B. um Quarz-, Feldspat- und Glimmerteilchen, die üblicherweise dadurch entfernt werden, dass eine Suspension des Erdalkali-Schichtsilicats durch einen Hydrozyklon geleitet werden.

Gegenstand der Erfindung ist auch ein mineralisches Bindemittelsystem, insbesondere ein Trockenmörtelsystem, enthaltend ein quellfähiges Schichtsilicat gemäß Anspruch 1. Zu den mineralischen Bindemittelsystemen gehören, ohne darauf beschränkt zu sein, Baustoffgemische, wie Mörtel, Estriche, Putze und Baukleber.

Gegenstand der Erfindung ist ferner die Verwendung des erfindungsgemäßen quellfähigen Schichtsilicats in die Quellfähigkeit herabsetzenden Medien. Dabei kann das erfindungsgemäße quellfähige Schichtsilicat Teil einer Pulvermischung sein, oder aber separat in Pulver- oder vordispergierter Form zu dem Medium unter Einwirkung von Scherkräften zugegeben werden.

Eine besondere Verwendung besteht in der Steuerung der Rheologie und/und der Stabilität von pulverförmigen und/oder flüssigen Medien mit einem Gehalt an Elektrolyten und/oder Tensiden.

Bei den Elektrolyten handelt es sich üblicherweise um lösliche Verbindungen von zwei- oder mehrwertigen Metallen, oder um Gemische aus löslichen und unlöslichen Verbindungen von zwei- oder mehrwertigen Metallen, wie Calciumcarbonat und Calciumhydroxid.

Eine besondere Verwendung liegt in der Steuerung der Rheologie und der Stabilität von pulverförmigen mineralischen Bindemittelsystemen, wie Systemen auf Basis von hydraulisch härtenden Bindemitteln, wobei das Schichtsilicat entweder Bestandteil einer Pulvermischung ist oder aus einer wässrigen Suspension unter Anwendung von Scherkräften mit den pulverförmigen mineralischen Bindemittelsystemen vermischt wird.

Auch das Absacken eines Putzes an der Wand kann durch Einsatz des erfindungsgemäßen Schichtsilicats verhindert und somit das Standvermögen erhöht werden.

Eine weitere besondere Verwendung liegt in der Steuerung der Rheologie und der Stabilität von Suspensionen und/oder Lösungen mit zwei- oder mehrwertigen Kationen (z. B. Suspensionen und/oder Lösungen von Calciumhydroxid (wie z.B. Kalkmilch), Calciumcarbonat oder ähnlichen calciumreichen Verbindungen).

Ein weiterer erfindungsgemäßer Vorteil besteht in der Verhinderung bzw. Verringerung der Synerese, insbesondere unter Transportbedingungen, sowie der Verbesserung der (Wieder-)Aufrührbarkeit in den vorgenannten Systemen und Medien, wie dies ansonsten nur durch organische Netzmittel erzielt werden kann.

Das Quellvolumen wurde wie folgt gemessen: Ein graduierter 100 ml - Messzylinder wird mit 100 ml destillierten Wasser gefüllt, 2g (atro) des zu messenden Schichtsilicats werden langsam und portionsweise je etwa 0,1 bis 0,2g mit einem Spatel auf die Oberfläche des Wassers gegeben. Man wartet das Absinken der zugegebenen Portion ab, bevor die nächste Portion zugegeben wird. Nachdem die 2g zugegeben und auf den Grund des Messzylinders abgesunken sind, wird nach einer Stunde die Höhe der aufgequollenen Substanz, die sie im Messzylinder einnimmt, in mm/2g abgelesen.

Die erfindungsgemäßen Schichtsilicate sind in den folgenden Beispielen in nicht einschränkender Weise näher erläutert.

Beispiel 1

Verwendet wird ein kommerziell erhältlicher, mit Soda aktivierter Ca-Bentonit (Handelsprodukt Tixoton® CV 15 der Firma Süd Chemie AG)(Probe A). Für dieses Material ergibt sich aus der Laser-(Malvern-)Pulveranalyse folgende Korngrößenverteilung:

| | |
|---|---|
| Rückstand auf 20 $\mu$m: | 60% |
| Rückstand auf 40 $\mu$m: | 40,8% |

Der mittlere Korndurchmesser beträgt damit etwa 30 $\mu$m. Ein Teil dieses Materials wird anschließend einer weiteren Vermahlung unterworfen (Probe C). Die Korngrößenverteilung dieses Materials ist (gemessen im selben Lasergerät):

| | |
|---|---|
| Rückstand auf 20 $\mu$m: | 10,53% |
| Rückstand auf 40 $\mu$m: | 0,38% |

Der mittlere Korndurchmesser beträgt etwa 4,5 $\mu$m.

Das Quellvolumen der Probe C beträgt 36 ml. Das Quellvolumen wurde wie vorstehend angegeben gemessen:

Versuch 1a):

Beide Proben (A und C) werden zunächst in Wasser dispergiert, und die Viskosität der 8%-igen Suspension wird in einem Brookfield-Rheometer vermessen. Testmethode: In einem 250 ml Becherglas werden 92g Leitungswasser vorgelegt. 8g Bentonit (lufttrocken) werden innerhalb von 60 sec unter Rühren im Labordissolver (940 UpM) zugegeben. Anschließend wird noch 10 min bei 2800 UpM nachgerührt. Die Messung der Viskosität erfolgt nach 1 h Quellzeit am Rheometer (Brookfield). Die erhaltenen Ergebnisse sind in Tabelle I angegeben:

Tabelle I

| Probe A | Probe A | Probe C | Probe C |
|---|---|---|---|
| Brookfield 10 UpM | Brookfield 100 UpM | Brookfield 10 UpM | Brookfield 100 UpM |
| Pa•s | Pa•s | Pa•s | Pa•s |
| 2200 | 310 | 2120 | 275 |

Im Testsystem Wasser allein ist zwischen dem Verdickungseffekt des Standardmaterials (A) und der nachvermahlenen Probe (C) kein Unterschied festzustellen.

Versuch 1b):

Wird die Fließgrenze mit der "Kugelharfe" nach Soos gemessen (Fließgrenze in $N/m^2$ an einer 6%-igen Suspension gemäß DIN 4126), so ergeben sich hier überraschenderweise deutliche Unterschiede.
Es wurde folgende Messmethode angewendet:
2 Liter Leitungswasser werden in einem 5 Liter-Behälter vorgelegt. 120g Schichtsilicat werden bei 940 UpM innerhalb von 60 sec mit einem Labordissolver eingerührt. Danach wird weitere 5 min bei 2800 UpM gerührt. Die Suspension wird dann in einen verschließbaren Behälter gegeben. Nach 1 h bzw. 24 h Quellzeit wird 1 Liter der Suspension in einen Kunststoffbehälter gefüllt und mit der Hand kurz aufgerührt. Nach 1 min Standzeit werden dann die Prüfkugeln mittels des Kugelharfengeräts in die Suspension eingetaucht. Die erste durchhängende Kugel ist maßgebend für die Fließgrenze (DIN 4126 Anhang B).
Die Ergebnisse sind in Tabelle II angegeben.

Tabelle II

| Probe A | Probe A | Probe C | Probe C |
|---|---|---|---|
| Fließgrenze nach 1 h | Fließgrenze nach 24 h | Fließgrenze nach 1 h | Fließgrenze nach 24 h |
| Kugel Nr. 4 | Kugel Nr. 6 | Kugel Nr. 5 | Kugel Nr. 8 |
| 18,1 $N/m^2$ | 35,9 $N/m^2$ | 25,2 $N/m^2$ | 49,9 $N/m^2$ |

Der Aufbau einer Fließgrenze erfolgt mit der nachvermahlenen Probe (C) schneller und ergibt höhere Werte. Diese Eigenschaft der Fließgrenze ist immer dann von Bedeutung, wenn entweder schwere Bestandteile eines fließfähigen wässrigen Systems am Absetzen gehindert werden sollen (Verfüllbeton) oder aber eine Standfestigkeit (z. B. eines Grundputzes an einer Mauer) verlangt wird.

Beispiel 2

Der Versuch der Fließgrenzenbestimmung von Beispiel 1b wird wiederholt, wobei jetzt aber statt des Schichtsilicats eine Mischung aus 95 Gew.-Teilen Schichtsilicat und 5 Gew.-Teilen Zement (CEM II/A - L 32,5 R) eingesetzt wird. Die Fließgrenze wird dabei in einem Bohlin-Rheometer CS 50 bestimmt: Probe B ist das Standardmaterial (A) mit 5 Gew.-% Zement; Probe D entspricht Probe C mit 5% Zement. Aus Tabelle III ist deutlich zu erkennen, dass bei Anwesenheit von Zement die feine Probe D (aus C) eine wesentlich bessere Wirksamkeit ergibt.

Tabelle III

| Probe B | Probe B | Probe D | Probe D |
|---|---|---|---|
| Fließgrenze nach 1 h | Fließgrenze nach 24 h | Fließgrenze nach 1 h | Fließgrenze nach 24 h |

(fortgesetzt)

| Probe B | Probe B | Probe D | Probe D |
|---------|---------|---------|---------|
| 27,6 Pa•s | 67,6 Pa•s | 42,5 Pa•s | 113,0 Pa•s |

Beispiel 3

Der Versuch von Beispiel 2 wird wiederholt mit einer Mischung Zement: Schichtsilicat = 85:15. Die Trockenmischung wird mit Leitungswasser im Gewichtsverhältnis 1:1 angesetzt. 100g Wasser werden im Becherglas vorgelegt, und 100g der Mischungen werden innerhalb von 120 sec unter Rühren im Labordissolver bei 940 UpM zugegeben. Danach wird weitere 5 min bei 2800 UpM gerührt. Die Proben werden dann nach einer Quellzeit von 15 min bzw. 30 min im Bohlin-Rheometer vermessen. Vor jeder Messung wird die Probe vorsichtig mit dem Spatel aufgerührt. Probe E = 15 Gew.-Teile Probe A + 85 Gew.-Teile Zement; Probe F = 15 Gew.-Teile Probe C + 85 Gew.-Teile Zement; Die Ergebnisse sind in Tabelle IV angegeben:

Tabelle IV

| Probe E | Probe E | Probe F | Probe F |
|---------|---------|---------|---------|
| Fließgrenze nach 15 min. | Fließgrenze nach 30 min. | Fließgrenze nach 15 min. | Fließgrenze nach 30 min. |
| 9,22 Pa•s | 7,58 Pa•s | 10,9 Pa•s | 12,6 Pa•s |

Trotz der hohen Zementanteile ist auch hier wieder eindeutig sichtbar, dass das erfindungsgemäße Material wirksamer ist und höhere Fließgrenzen aufbaut. Diese Fließgrenze ist bei der erfindungsgemäßen Probe (F) auch stabil und verbessert sich sogar noch etwas, was auf ein Nachquellen hindeutet, während das Standardmaterial (E) schon flockt und einen Abbau der Fließgrenze zeigt.

Beispiel 4

In diesem Beispiel wird der Einfluss der Feinheit auf die Dispergierbarkeit der verwendeten Materialien in einer schon vorliegenden Zementsuspension geprüft. Je 4g der Proben A und C werden dazu in eine vorgelegte Zementsuspension (50 g Zement in 50g Wasser) innerhalb von 60 sec eingerührt (940 UpM) und dann weitere 5 min nachgerührt (1865 UpM). Die Messung der Proben erfolgt wieder im Bohlin-Rheometer nach einer Quellzeit von 15 bzw. 30 min.

Probe G = 4g Probe A in 100g Zement + Wasser,
Probe H = 4g Probe C in 100g Zement + Wasser.

Die Ergebnisse sind in Tabelle V angegeben.

Tabelle V

| Probe G | Probe G | Probe H | Probe H |
|---------|---------|---------|---------|
| Fließgrenze nach 15 min. | Fließgrenze nach 30 min. | Fließgrenze nach 15 min. | Fließgrenze nach 30 min. |
| 10,9 Pa•s | 10,9 Pa•s | 12,5 Pa•s | 12,6 Pa•s |

Die erfindungsgemäßen Materialien sind auch in diesem System (4% Schichtsilicat bezogen auf Zement) dem Standardmaterial überlegen.

Beispiel 5

Nach diesem Beispiel wurde die Beeinflussung/Reduzierung des Rückprallanteils eines Spritzbetons untersucht.

Der Rückprallanteil ist von der Spritzbetonkonsistenz an der Auftragsfläche abhängig. Diese wird wiederum durch den Kornaufbau des Fein- und Grobgutzuschlags sowie durch die Menge und Fließfähigkeit des Zementleims beeinflusst. Der auf die Auftragsfläche auftreffende Beton sollte möglichst plastisch sein, damit sich die auftreffenden Zuschlagskörner gut einbetten können. Dabei darf aber das Eigengewicht nicht größer sein als die Zugfähigkeit (innere Kohäsion) des Spritzbetons bzw. die Haftzugfestigkeit an der Grenzfläche Spritzbeton/Untergrund oder gar die Zugfestigkeit des Untergrunds. Sowohl unzureichendes Klebevermögen als auch innere Kohäsion hätte sonst einen Rückprall zur Folge. Daher ist ein thixotropes Fließverhalten mit schnellem Aufbau einer ausgeprägten Fließgrenze anzustreben. Als Feinstmörtel wurde verwendet:

```
Trockenmörtel:   500g CEM I 42,5 R + 150g Quarzsand 0/0,5 +

pulverförmiges Schichtsilicat
```

```
Anmachwasser:    250g Gesamtwasser + flüssiges Schichtsilicat
```

Die jeweiligen Dosiermengen der Probe C wurden im Vorfeld den Zementproben mittels Quirl zugegeben und etwa 2 min homogenisiert. Infolge der unterschiedlich hohen Dosiermengen verändert sich auch der Wasseranspruch, so dass beim Spritzen nach den Spritzversuchen mit konstantem W/Z (Wasser/Zement) Wert das Augenmerk auf eine gleiche Konsistenz des Spritzbetons gelegt wurde.

Bei diesen Versuchen wurden der Zuschlag "Eberstein[®]" (Dolomit Eberstein Neuper GmbH) GK 4mm; Sieblinie (A+B)/2; 3% Eigenfeuchte) und das Spritzbindmittel (Chronolith[®] ST, Fa. Heidelberger Zement) getrennt in den Zugabetrichter zweier Schneckendosierer geleert. Die Dosierung des Bindemittels erfolgte unmittelbar vor Spritzbeginn mit kalibrierten Schnecken, die so eingestellt waren, dass sich ein Bindemittelgehalt von 380 kg/m$^3$ ergab. Das Mischungsverhältnis Bindemittel/Zuschlag betrug 1: 4,88 und wurde für die gesamte Versuchsreihe konstant gehalten. Die Wasserzugabe (Druck: 7 bar) erfolgte an der Düse, welche vom Düsenführer durch ständiges Beobachten des Wasserdurchflussmessers auf den zuvor festgelegten W/Z Wert genau eingehalten werden konnte. Der Spritzdüsenabstand zur Auftragsfläche betrug in allen Versuchen 0,8 bis 1,0 m.

Zur Erfassung des Rückprallanteils wurde beim Applizieren des Spritzbetons in Holzkisten (30 x 40 x 10 cm; Spritzbetonschichtstärke = etwa 10 cm) der Rückprall durch eine auf dem Boden ausgebreitete Plane aufgefangen und gewogen. Die Masse des aufgespritzten Betons ergibt sich aus der Differenzwägung Masse Spritzbeton + Schalung abzüglich Masse der Schalung. Folglich errechnet sich der Rückprall in Prozent:

$$\text{Rückprall:} \quad \frac{\text{Masse }_{\text{Rückprall}}}{\text{Masse }_{\text{Spritzbeton}} + \text{Masse }_{\text{Rückprall}}} \times 100 \; [\%]$$

Die Ergebnisse sind in Tabelle VI angegeben:

Tabelle VI

| Schichtsilicat | Dosierung (Gew.-% des Zementgehalts) | Rückprallwert (%) | W/Z Wert |
|----------------|--------------------------------------|-------------------|----------|
| C | 0,0 | 30,2 | 0,47 |
| C | 1,0 | 23,8 | 0,47 |
| C | 1,6 | 22,8 | 0,47 |
| C | 2,0 | 27,8 | 0,47 |
| C | 1,0 | 21,6 | 0,48 |
| C | 1,6 | 14,8 | 0,51 |
| C | 2,0 | 15,4 | 0,52 |
| C | 0,0 | 25,1 | 0,51 |
| A | 1,0 | 25,2 | 0,46 |
| A | 2,0 | 30,4 | 0,44 |

(fortgesetzt)

| Schichtsilicat | Dosierung (Gew.-% des Zementgehalts) | Rückprallwert (%) | W/Z Wert |
|---|---|---|---|
| A | 2,0 | 22,3 | 0,53 |

Die Zugabe des erfindungsgemäßen pulverförmigem Schichtsilicats zeigt also eine gute Reduzierung des Rückprallanteils, was nur durch die schnelle Ausbildung einer Kartenhausstruktur, d. h. durch Delaminierung erreichte Fließgrenze/Thixotropie erklärt werden kann. Das Standard-Vergleichsmaterial A zeigt zwar ebenfalls eine gewisse Wirkung, diese ist aber deutlich schlechter als die mit dem erfindungsgemäßen Material erzielte.

Beispiel 6

Verwendet werden mit Soda aktivierte Ca-Bentonite (Tixoton®-Produkte der Firma Süd-Chemie AG analog Probe A aus Beispiel 1) mit unterschiedlicher Feinheit und Aufbereitung:

Probe A, Standardbentonit: Feinheit von 20% > 45 $\mu$m
Probe B, Bentonit mit feinster Vermahlung: Feinheit von 3% > 45 $\mu$m
Probe C, Bentonit mit feinster Vermahlung und Ausreinigung durch Sichtung: Feinheit von 1 % > 45 $\mu$m

Versuch 6a)

Die Proben A, B und C werden zunächst in Wasser dispergiert, und die Viskosität der 5%-igen Suspension wird mit einem Brookfield-Viskosimeter gemessen.

Testmethode: In einem 600 ml-Becherglas werden 190 g Leitungswasser vorgelegt. 10 g Bentonit (lufttrocken) werden innerhalb von 60 sec. unter Rühren mittels Labordissolver (930 UpM) zugegeben. Anschließend wird 15 min bei 2800 UpM gerührt. Die Messung der Viskosität erfolgt nach 1 Tag Quellzeit am Viskosimeter (Brookfield). Die erhaltenen Ergebnisse sind in Tabelle VII angegeben.

Tabelle VII

| | Viskosität, Brookfield 10 UpM (mPa•s) |
|---|---|
| Probe A | 3300 |
| Probe B | 3780 |
| Probe C | 4500 |

Versuch 6b) (30%-ige Ca(OH)$_2$-Suspension)

Rezeptur: 50 Teile Bentonit-Suspension, 5%-ig
20 Teile Wasser
30 Teile Löschkalk-Pulver (Ca(OH)$_2$)

In einem 600 ml-Becherglas werden 150 g Bentonitsuspension aus Versuch 6a vorgelegt und mit 60 g Wasser versetzt. Zur Homogenisierung wird 15 sec mit einem Labordissolver gerührt. Bei einer Nullprobe ohne Bentonit werden stattdessen 210 g Wasser vorgelegt. Danach werden 90 g Löschkalk-Pulver unter laufendem Dissolver portionsweise im Laufe von 5 min. zugegeben. Dabei wird die Rührgeschwindigkeit schrittweise erhöht und beträgt zuletzt 2800 UpM. Teilproben von 100 ml werden in einen 100 ml-Messzylinder aus Glas (Durchmesser 3 cm) gefüllt. Die Restproben werden in Kunststoffbehälter gefüllt.

Nach 1 Woche Standzeit wird die klare Flüssigkeitsabscheidung (Synerese) oben im Messzylinder abgelesen und bei den in Kunststoffbehältern gelagerten Proben die Viskosität (Brookfield) gemessen.

Aus Tabelle VIII ist deutlich zu erkennen, dass herkömmliche Bentonitprodukte (A) trotz einer gewissen Verdickungswirkung kaum einen stabilisierenden Effekt für die Ca(OH)$_2$-Suspension ergeben. Mit gleichen Mengen (2,5%) der feineren Bentonite (B und C) wird dagegen die Synerese deutlich verringert.

Tabelle VIII

|  | Synerese nach 1 Woche | Viskosität, Brookfield 10 UpM (mPa•s) |
|---|---|---|
| Nullprobe | 15% | 1520 |
| Probe A | 12% | 3020 |
| Probe B | 8% | 3740 |
| Probe C | 3,5% | 4280 |

Beispiel 7

Der Versuch von Beispiel 6 wird wiederholt, wobei jedoch die Konzentration der zu stabilisierenden Ca (OH)$_2$-Suspension 40% beträgt. Entsprechend dem geringeren Wassergehalt und der höheren Grundviskosität wird weniger Bentonit zur Stabilisierung eingesetzt, nämlich 0,75% (entsprechend 15% der 5%-igen Bentonit-Suspension aus Versuch 6a).

Rezeptur:   45 g (15%) Bentonit-Suspension, 5%-ig
135 g (45%) Wasser
120 g (40%) Löschkalk-Pulver (Ca(OH)$_2$)

Die Testmethode ist analog zu Versuch 6b, bei der Nullprobe werden 180 g Wasser vorgelegt. Die Ergebnisse in Tabelle IX belegen auch hier die überlegene Stabilisierungswirkung der feineren Bentonite (B und C).

Tabelle IX

|  | Synerese nach 1 Woche | Viskosität, Brookfield 10 UpM (mPa•s) |
|---|---|---|
| Nullprobe | 3,5% | 3640 |
| Probe A | 3% | 4160 |
| Probe B | 2% | 4340 |
| Probe C | 1% | 5320 |

Beispiel 8

Der Versuch aus Beispiel 7 wird wiederholt, wobei die Ansatzgröße 2 kg statt 300 g beträgt. Die Proben werden in verschlossene Kunststoffbehälter gefüllt und in einem Kraftfahrzeug 100 km transportiert (Anteile Stadtverkehr, Landstraße, Autobahn). Unter diesen Transportbedingungen ist die Flüssigkeitsabscheidung (Synerese) generell höher als bei ruhiger Lagerung in Glaszylindern.
Die Messung geschieht hier durch Abgießen und Auswiegen der klaren Flüssigkeit. Nach Zurückgießen wird die Aufrührbarkeit der sedimentierten Suspension qualitativ beurteilt.
Die Ergebnisse in Tabelle X zeigen, dass feine Bentonite im Vergleich zu Standardbentoniten nicht nur die Synerese unter Transportbedingungen reduzieren, sondern auch die anschließende Aufrührbarkeit verbessern.

Tabelle X

|  | Synerese nach 100 km Kfz-Transport | Aufrührbarkeit |
|---|---|---|
| Nullprobe | 15% | sehr schlecht |
| Probe A | 14% | schlecht |
| Probe B | 8% | gut |
| Probe C | 5% | sehr gut |

**EP 1 395 524 B1**

**Patentansprüche**

1. Quellfähiges Schichtsilicat auf Smektit- und/oder Sepiolith/Palygorskit-Basis zur Verwendung in die Quellfähigkeit herabsetzenden, zwei- oder mehrwertige Kationen enthaltenden Medien, **gekennzeichnet durch** ein Quellvolumen von etwa 5 bis 50 ml, gemessen **durch** Zugabe von 2g Schichtsilicat (atro) zu 100 ml Wasser, und **durch** eine Feinheit von maximal 10 Gew.-% > 20 $\mu$m, vorzugsweise von maximal 5% > 10 $\mu$m, gemessen als Trockensieb- rückstand.

2. Schichtsilicat nach Anspruch 1, **dadurch gekennzeichnet, dass** es ein Quellvolumen von etwa 10 bis 40 ml, insbesondere von etwa 20 bis 35 ml, gemessen durch Zugabe von 2g Schichtsilicat zu 100 ml Wasser aufweist.

3. Schichtsilicat nach Anspruch 1 oder 2, **dadurch gekennzeichnet, dass** es aus Bentonit, Montmorillonit, Hectorit, Attapulgit bzw. Palygorskit, Saponit, Beidellit, Sauconit natürlichen oder synthetischen Ursprungs oder deren Gemischen gegebenenfalls mit einem Anteil an schichtförmigen Extendersilicaten wie Vermiculit, Illit, Glimmer, Pyrophillit, Talk, Kaolin ausgewählt ist.

4. Schichtsilicat nach einem der Ansprüche 1 bis 3, **dadurch gekennzeichnet, dass** es mehr als etwa 70 Gew.-%, vorzugsweise mehr als etwa 80 Gew.-%, insbesondere mehr als etwa 85 Gew.-% Montmorillonit enthält.

5. Schichtsilicat nach einem der Ansprüche 1 bis 4, **gekennzeichnet durch** den Zusatz von pulverförmigen alkalihaltigen Substanzen.

6. Verfahren zur Herstellung des Schichtsilicats nach einem der Ansprüche 1 bis 5, **gekennzeichnet durch** alkalische Aktivierung eines Erdalkali-Schichtsilicats, Trocknen und Vermahlen des Materials mit überlagerter Sichtung.

7. Verfahren zur Herstellung des Schichtsilicats nach einem der Ansprüche 1 bis 5, **gekennzeichnet durch** alkalische Aktivierung eines in einem wässrigen Medium suspendierten Erdalkali-Schichtsilicats, Entfernung der groben Zuschlagstoffe und Sprühtrocknung der verdünnten Suspension, sowie gegebenenfalls **durch** Mahlung und Sichtung der sprühgetrockneten Teilchen, falls diese die in Anspruch 1 angegebene Kornfeinheit noch nicht erreicht haben.

8. Mineralisches Bindemittelsystem, insbesondere Trockenmörtelsystem, enthaltend ein quellfähiges Schichtsilicat gemäß einem der Ansprüche 1 bis 5.

9. Mineralisches Bindemittelsystem nach Anspruch 8, **dadurch gekennzeichnet, dass** es 0,1 bis 5 Gew.-%, insbesondere 0,1 bis 3 Gew.-%, bevorzugt 0,1 bis 1,5 Gew.-%, und besonders bevorzugt 0,2 bis 1,0 Gew.-% an quellfähigem Schichtsilicat bezogen auf die Gesamtzusammensetzung enthält.

10. Verwendung des quellfähigen Schichtsilicats nach einem der Ansprüche 1 bis 5 bzw. hergestellt nach Anspruch 6 oder 7 in die Quellfähigkeit herabsetzenden, insbesondere in zwei- oder mehrwertige Kationen enthaltenden Medien.

11. Verwendung nach Anspruch 10 zur Steuerung der Rheologie und/oder der Stabilität von pulverförmigen und/oder flüssigen/pastösen Medien mit einem Gehalt an Elektrolyten und/oder Tensiden.

12. Verwendung nach Anspruch 11, **dadurch gekennzeichnet, dass** die Elektrolyte lösliche Verbindungen von zwei- oder mehrwertigen Metallen darstellen.

13. Verwendung nach einem der Ansprüche 10 bis 12 zur Steuerung der Rheologie und/oder der Stabilität von pulverförmigen mineralischen Bindemittelsystemen, wie Systemen auf Basis von hydraulischen, latent hydraulischen oder an Luft härtenden Bindemitteln, wobei das Schichtsilicat entweder Bestandteil einer Pulvermischung ist oder aus einer wässrigen Suspension unter Anwendung von Scherkräften mit den pulverförmigen mineralischen Bindemittelsystemen vermischt wird.

14. Verwendung des quellfähigen Schichtsilicats nach einem der Ansprüche 1 bis 5 bzw. hergestellt nach Anspruch 6 oder 7 in Suspensionen und/oder Lösungen mit zwei- oder mehrwertigen Kationen, insbesondere Suspensionen und/oder Lösungen von Calciumhydroxid, Calciumcarbonat oder ähnlichen calciumreichen Verbindungen zur Stabilisierung und/oder Viskositätserhöhung.

15. Verwendung der Schichtsilicate gemäß Anspruch 1 zur Steuerung der Rheologie und der Stabilität in pulverförmigen und/oder flüssigen Systemen, mit einem Anteil an Elektrolyten wie Abwasserbehandlungsmittel (wie z.B. Ca(OH)$_2$-Suspensionen), Haushaltsreiniger, Kosmetika, Spachtelmassen, Schmiermittel, Fugenmassen etc., wobei das neuartige Schichtsilicat entweder ein Bestandteil einer Pulvermischung sein kann oder aber zu einer Lösung bzw. Suspension separat unter Scherkräften zugegeben werden kann.

16. Verwendung des quellfähigen Schichtsilicats nach einem der Ansprüche 1 bis 5 bzw. hergestellt nach Anspruch 6 oder 7 zur Steuerung der Rheologie und der Stabilität speziell von pulverförmigen mineralischen Bindemittelsystemen wie z.B. Systemen auf Basis von hydraulisch härtenden Bindemitteln wie Zement oder Kalk-Gips, Anhydrit etc., wobei das neuartige Schichtsilicat entweder Bestandteil einer Pulvermischung sein kann oder aber zu einer Lösung bzw. Suspension separat unter Scherkräften zugegeben werden kann.

17. Verwendung des quellfähigen Schichtsilicats nach einem der Ansprüche 1 bis 5 bzw. hergestellt nach Anspruch 6 oder 7 zur Verhinderung bzw. Verringerung der Synerese insbesondere unter Transportbedingungen und/oder der Verbesserung der Aufrührbarkeit bzw. Wiederaufrührbarkeit in zwei- oder mehrwertige Kationen enthaltenden Medien bzw. Systemen.

## Claims

1. A swellable layered silicate with a smectite and/or sepiolite/palygorskite basis for use in media containing di or polyvalent cations that reduce swelling capacity, **characterised by** a swelling volume of approximately 5 to 50 ml, measured by addition of 2 g of layered silicate (absolutely dry) to 100 ml water, and by a fineness of at maximum 10 % by weight > 20 $\mu$m, preferably of at maximum 5 % > 10 $\mu$m, measured as a dry sieve residue.

2. A layered silicate according to Claim 1, **characterised in that** it has a swelling volume of approximately 10 to 40 ml, in particular of approximately 20 to 35 ml, measured by addition of 2 g of layered silicate to 100 ml water.

3. A layered silicate according to Claim 1 or 2, **characterised in that** it is selected from bentonite, montmorillonite, hectorite, attapulgite or palygorskite, saponite, beidellite, sauconite of natural or synthetic origin or mixtures thereof, optionally with a content of layered extender silicates, such as vermiculite, illite, mica, pyrophyllite, talc and kaolin.

4. A layered silicate according to any one of Claims 1 to 3, **characterised in that** it contains more than approximately 70 % by weight, preferably more than approximately 80 % by weight, in particular more than approximately 85 % by weight of montmorillonite.

5. A layered silicate according to any one of Claims 1 to 4, **characterised by** the addition of a powdered alkali-containing substance.

6. A method of preparing the layered silicate according to any one of Claims 1 to 5, **characterised by** alkaline activation of an alkaline earth layered silicate, drying and grinding of the material with superimposed screening.

7. A method of preparing the layered silicate according to any one of Claims 1 to 5, **characterised by** alkaline activation of an alkaline earth layered silicate, suspended in an aqueous medium, removal of the coarse aggregates and spray drying of the diluted suspension, as well as optionally by grinding and screening the spray-dried particles, if they have not yet attained the grain fineness indicated in Claim 1.

8. A mineral binder system, in particular a dry mortar system, containing a swellable layered silicate according to any one of Claims 1 to 5.

9. A mineral binder system according to Claim 8, **characterised in that** it contains 0.1 to 5 % by weight, in particular 0.1 to 3 % by weight, preferably 0.1 to 1.5 % by weight, and especially preferably 0.2 to 1.0 % by weight of swellable layered silicate in relation to the total composition.

10. Use of the swellable layered silicate according to any one of Claims 1 to 5 or prepared according to Claim 6 or 7, in media containing di or polyvalent cations that reduce swelling capacity.

11. Use according to Claim 10 for controlling the rheology and/or stability of powdered and/or fluid/pasty media with a content of electrolytes and/or surfactants.

**12.** Use according to Claim 11, **characterised in that** the electrolytes represent soluble compounds of di or polyvalent metals.

**13.** Use according to any one of Claims 10 to 12 for controlling the rheology and/or stability of powdered mineral binder systems, such as systems based on hydraulic, latent hydraulic or air-hardening binders, wherein the layered silicate is a either a constituent of a powder mixture or is mixed with the powdered mineral binder systems from an aqueous suspension with application of shearing forces.

**14.** Use of the swellable layered silicate according to any one of Claims 1 to 5 or prepared according to Claim 6 or 7 in suspensions and/or solutions with di or polyvalent cations, in particular suspensions and/or solutions of calcium hydroxide, calcium carbonate or similar pure calcium compounds for stabilisation and/or increasing viscosity.

**15.** Use of the layered silicates according to Claim 1 for controlling the rheology and stability in powdered and/or fluid systems, with a content of electrolytes as effluent-treatment agents (for example, Ca(OH)$_2$ suspensions), domestic cleansers, cosmetics, fillers, lubricants, jointing compounds, etc., wherein the novel layered silicate may either be a constituent of a powder mixture or else may be added separately to a solution or suspension with application of shearing forces.

**16.** Use of the swellable layered silicate according to any one of Claims 1 to 5 or prepared according to Claim 6 or 7, for controlling the rheology and stability especially of powdered mineral binder systems, for example systems based on hydraulically hardening binders, such as cement or lime gypsum, anhydrite, etc., wherein the novel layered silicate may either be a constituent of a powder mixture or else may be added separately to a solution or suspension with application of shearing forces.

**17.** Use of the swellable layered silicate according to any one of Claims 1 to 5 or prepared according to Claim 6 or 7, for preventing or reducing syneresis, in particular under transportation conditions and/or improving the capacity to be stirred or re-stirred into media or systems containing di or polyvalent cations.

**Revendications**

**1.** Silicate en couche expansif à base de smectite et/ou de sépiolithe/palygorskite pour une utilisation dans des milieux abaissant l'expansibilité et contenant des cations bivalents ou multivalents, **caractérisé par** un volume de gonflement d'environ 5 à 50 ml, mesuré par l'addition de 2 g de silicate en couche (atro) à 100 ml d'eau, et par une finesse d'au maximum 10 % en poids > 20 μm, de préférence de maximum 5 % en poids > 10 μm, mesuré comme résidu de tamisage à sec.

**2.** Silicate en couche selon la revendication 1, **caractérisé en ce qu'**il présente un volume de gonflement d'environ 10 à 40 ml, en particulier d'environ 20 à 35 ml, mesuré par l'addition de 2 g de silicate en couche à 100 ml d'eau.

**3.** Silicate en couche selon la revendication 1 ou 2, **caractérisé en ce qu'**il est sélectionné à partir de bentonite, de montmorillonite, d'hectorite, d'attapulgite ou de palygorskite, de saponite, de beidellite, de sauconite d'origine naturelle ou synthétique ou de leurs mélanges éventuellement avec une fraction de silicates extendeurs en forme de couche tels que vermiculite, illite, mica, pyrophillite, talc, kaolin.

**4.** Silicate en couche selon l'une quelconque des revendications 1 à 3, **caractérisé en ce qu'**il contient plus d'environ 70 % en poids, de préférence plus d'environ 80 % en poids, en particulier plus d'environ 85 % en poids de montmorrillonite.

**5.** Silicate en couche selon l'une quelconque des revendications 1 à 4, **caractérisé par** l'addition de substances alcalines pulvérulentes.

**6.** Procédé pour la fabrication du silicate en couche selon l'une quelconque des revendications 1 à 5, **caractérisé par** une activation alcaline d'un silicate en couche alcalino-terreux, un séchage et un broyage du matériau avec tri superposé.

**7.** Procédé pour la fabrication du silicate en couche selon l'une quelconque des revendications 1 à 5, **caractérisé par** une activation alcaline d'un silicate en couche alcalino-terreux mis en suspension dans un milieu aqueux, une élimination des granulats grossiers et un séchage par pulvérisation de la suspension diluée, et éventuellement par un broyage et tri des particules séchées par pulvérisation, si celles-ci n'ont pas encore atteint la finesse de grain indiquée à la revendication 1.

8. Système de liant minéral, en particulier système de mortier sec, contenant un silicate en couche expansif selon l'une quelconque des revendications 1 à 5.

9. Système de liant minéral selon la revendication 8, **caractérisé en ce qu'**il contient 0,1 à 5 % en poids, en particulier 0,1 à 3 % en poids, de préférence 0,1 à 1,5 % en poids, et avec une préférence particulière de 0,2 à 1,0 % en poids de silicate de couche expansif par rapport à la composition globale.

10. Utilisation du silicate en couche expansif selon l'une quelconque des revendications 1 à 5 ou fabriqué selon la revendication 6 ou 7, dans des milieux réduisant l'expansibilité, contenant en particulier des cations bivalents ou polyvalents.

11. Utilisation selon la revendication 10 pour le contrôle de la rhéologie et/ou de la stabilité de milieux pulvérulents et/ou liquides/pâteux avec une teneur en électrolytes et/ou agents tensioactifs.

12. Utilisation selon la revendication 11, **caractérisé en ce que** les électrolytes présentent des composés solubles de métaux bivalents ou multivalents.

13. Utilisation selon l'une quelconque des revendications 10 à 12 pour le contrôle de la rhéologie et/ou de la stabilité de systèmes de liant minéraux pulvérulents, tels que des systèmes à base de liants hydrauliques, hydrauliques de façon latente ou durcissant à l'air, le silicate en couche étant soit un composant d'un mélange de poudre soit étant mélangé à partir d'une suspension aqueuse avec l'utilisation des forces de cisaillement, avec les systèmes de liant minéraux pulvérulents.

14. Utilisation du silicate en couche expansif selon l'une quelconque des revendications 1 à 5 ou fabriqué selon la revendication 6 ou 7 dans des suspensions et/ou des solutions avec des cations bivalents ou multivalents, en particulier des suspensions et/ou des solutions d'hydroxyde de calcium, de carbonate de calcium ou de composés riches en calcium identiques pour la stabilisation et/ou l'augmentation de la viscosité.

15. Utilisation des silicates en couche selon la revendication 1 pour le contrôle de la rhéologie et de la stabilité dans des systèmes pulvérulents et/ou liquides, avec une fraction d'électrolytes comme des agents de traitement d'eaux usées (comme par exemple des suspensions de $Ca(OH)_2$), des nettoyants ménagers, des produits de beauté, des mastics, des lubrifiants, des pâtes pour joints, etc., le silicate de type nouveau en couche pouvant être un composant d'un mélange de poudre ou pouvant être ajouté séparément à une solution ou une suspension sous l'effet de forces de cisaillement.

16. Utilisation du silicate en couche expansif selon l'une quelconque des revendications 1 à 5 fabriqué selon la revendication 6 ou 7 pour le contrôle de la rhéologie et de la stabilité notamment de systèmes de liants minéraux pulvérulents, par exemple des systèmes à base de liants durcissant de façon hydraulique tels que le ciment ou le gypse, l'anhydrite, etc., le silicate en couche de type nouveau pouvant être un composant d'un mélange de poudre ou pouvant être ajouté à une solution ou suspension séparément sous l'effet de forces de cisaillement.

17. Utilisation du silicate en couche expansif selon l'une quelconque des revendications 1 à 5 ou fabriqué selon la revendication 6 ou 7, pour empêcher ou réduire la synérèse en particulier en ce qui concerne les conditions de transport et/ou pour améliorer la possibilité de remuer ou de remuer à nouveau des milieux ou des systèmes contenant des cations bivalents ou multivalents.